# EUROPEAN PATENT SPECIFICATION

(11) **EP 3 409 196 B1**
(45) Date of publication and mention of the grant of the patent: **13.08.2025**
(21) Application number: 18175042.3
(22) Date of filing: 30.05.2018
(51) Int. Cl.: A61B 5/08, A61B 5/087

(54) **RESPIRATORY PRESSURE SENSOR**
ATEMDRUCKSENSOR
CAPTEUR DE PRESSION RESPIRATOIRE

(30) Priority: 31.05.2017 JP 2017108749
(43) Date of publication of application: 05.12.2018
(73) Proprietor: Nihon Kohden Corporation, Shinjuku-ku Tokyo (JP)
(72) Inventor: YAMAGISHI, Anna, Tokorozawa-shi, Saitama (JP); TSUJI, Makoto, Tokorozawa-shi, Saitama (JP); HIGASHI, Kazuri, Tokorozawa-shi, Saitama (JP); SUGIYAMA, Takayuki, Tokorozawa-shi, Saitama (JP)
(74) Representative: Grünecker Patent- und Rechtsanwälte PartG mbB

(56) References cited:
- EP-A1- 2 767 226
- US-A1- 2008 011 296
- US-A1- 2008 243 020
- US-A1- 2009 143 996
- US-A1- 2013 331 726
- US-A1- 2015 165 145
- US-A1- 2016 310 047
- US-A1- 2017 119 252

## Description

### BACKGROUND

The present disclosure relates to a respiratory pressure sensor.

JP-T-2000-500379 discloses a respiration monitoring device in which a pressure sensor fluid-connected to the nose of a patient and a temperature sensor operating to generate a signal indicating a temperature inside the nose of the patient and in the vicinity of the mouth of the patient are used in combination for diagnosis of sleep disorder.

When the patient falls into a sleep state, the respiration monitoring device in JP-T-2000-500379 monitors a respiration pattern of the patient for a predetermined period. Then, outputs of the pressure sensor and the temperature sensor are analyzed.

The respiration monitoring device determines whether the patient's respiration has a normal respiration pattern or exhibits respiration disorder.

Real-time analysis of a detection result is required for postoperative respiration management etc. of a patient in a hospital more than before. However, the respiration monitoring device in JP-T-2000-500379 has a configuration in which the frequency etc. of appearance of abnormal respiration is analyzed after respiration monitoring. Due to the configuration, the respiration monitoring device in JP-T-2000-500379 cannot not analyze the detection result in real time. US 2009/0143996 A1 describes an airway sensor.

An object of the present disclosure is to provide a respiratory pressure sensor for sensing intraoral or intranasal pressure in order to enable real-time analysis of a detection result.

### SUMMARY

A respiratory pressure sensor includes an input that receives respiratory gas from at least one of a nasal cavity or an oral cavity of a subject, a pressure sensor that detects respiratory pressure of the respiratory gas received from the input, a signal processor that performs signal processing on the respiratory pressure detected by the pressure sensor and generates respiration data, and an output that outputs the respiration data to an external device.

According to the aforementioned configuration, the respiration data about the respiratory gas acquired from the subject are generated, and the respiration data are outputted to the external device. Thus, a detection result of the respiratory gas can be analyzed in real time.

According to the respiratory pressure sensor of the present disclosure, the detection result can be analyzed in real time. The current invention is defined by the claims.

### BRIEF DESCRIPTION OF DRAWINGS

Fig. 1 is a conceptual explanatory view illustrating respiratory pressure sensor and an external device connected to the respiratory pressure sensor.
Fig. 2 is an explanatory view schematically illustrating the respiratory pressure sensor.
Fig. 3 is an explanatory view illustrating an example of an internal structure of a signal processor.

### DETAILED DESCRIPTION OF EMBODIMENTS

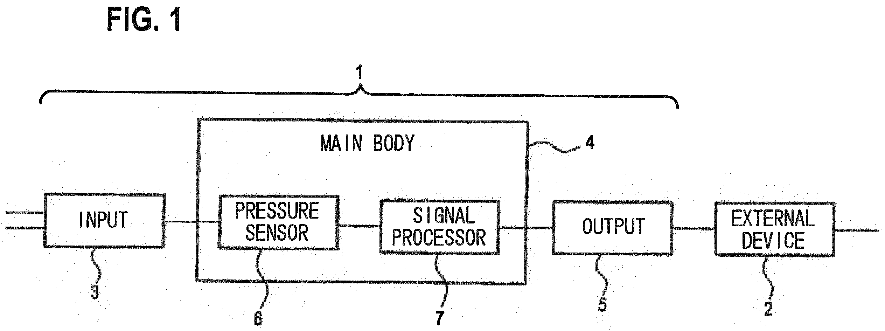

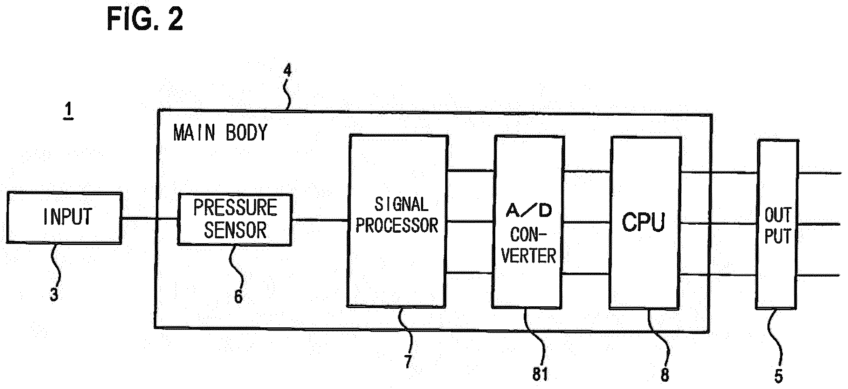

An example of an embodiment of a respiratory pressure sensor of the present disclosure will be described below with reference to the drawings. Fig. 1 is a conceptual explanatory view illustrating the respiratory pressure sensor 1 of the embodiment of the present disclosure, and an external device 2 connected to the respiratory pressure sensor 1. Fig. 2 is an explanatory view schematically illustrating the respiratory pressure sensor 1.

As shown in Fig. 1 and Fig. 2, the respiratory pressure sensor 1 is a sensor that outputs respiration data to the external device 2. The respiratory pressure sensor 1 has an input 3, a main body 4 and an output 5, as shown in Fig. 1.

The input 3 is a portion that acquires respiratory gas from at least one of the inside of the mouth and the inside of the nose of a subject. As an example, the input 3 can be used as a canula adaptor to which a canula attached to at least one of the mouth and the nose can be connected.

The respiratory gas includes expired gas and inspired air of the subject. That is, the respiratory gas is vital signs information useful for analyzing presence/absence of apnea, hypopnea or upper airway obstruction. The respiratory gas acquired by the input 3 includes a detection result about the respiratory gas, including a volume of the expired gas, a volume of the inspired air, detection times of the expiration and the inspiration, etc. The input 3 introduces the acquired respiratory gas into the main body 4.

The main body 4 is a device that receives the respiratory gas from the input 3 and generates respiration data based on the respiratory gas. The main body 4 has a pressure sensor 6, and a signal processor 7.

The pressure sensor 6 is configured to receive the respiratory gas from at least one of the inside of the mouth and the inside of the nose of the subject from the input 3, and detect a change in respiratory pressure of the received respiratory gas. The pressure sensor 6 is configured to output the detected respiratory pressure to the signal processor 7. For example, the respiratory pressure output from the pressure sensor is a voltage value.

The signal processor 7 is configured to apply signal processing to the respiratory pressure detected by the pressure sensor 6 and generate respiration data which is, for example, analog data. According to an example of the signal processing, a signal waveform corresponding to the respiratory pressure is generated.

As shown in Fig. 2, the main body 4 may further includes a CPU (Central Processing Unit) 8 serving as a controller, and a memory. The memory is not shown in Fig. 2. The memory may be configured to store computer-readable commands (i.e. programs). For example, the memory may be constituted by an ROM (Read Only Memory) in which various programs etc. have been stored, an RAM (Random Access Memory) having a plurality of work areas in which the various programs etc. executable by the CPU 8 can be stored, etc. The CPU 8 may be configured to expand, onto the RAM, a program designated from the various programs preinstalled in the ROM, and execute various processings in cooperation with the RAM. The CPU 8 can acquire the respiration data from the signal processor 7, and perform data processing and communication control etc. with the external device 2 to output the respiration data to the external device 2.

As shown in Fig. 2, the main body 4 may include an A/D converter 81 and the CPU 8. The A/D converter 81 is configured to receive the respiration data generated by the signal processor 7 and digitally convert the respiration data.

The output 5 is configured to receive the respiration data from the main body 4 and output the respiration data to the external device 2. The main body 4 and the output 5 are connected to each other through an electric cable etc. The output 5 is configured to be able to output data in accordance with system specification of the external device. For example, the output 5 may a connector that can be connected to a patient monitor or display that is an example of the external device 2, or a connector terminal that can be connected to the external device 2.

The respiration data outputted to the external device 2 includes identification information for identifying whether the external device 2 can recognize the respiration data or not.

The identification information is included in the respiration data outputted to the external device 2 and may be stored at any place in the respiratory pressure sensor 1. For example, the identification information may be held in the memory or may be held in the signal processor 7. Alternatively, the identification information may be held in a memory (not shown) mounted in the output 5.

For example, the identification information that is recognizable by the external device 2 may be identification information for distinguishing the respiration data received by the respiratory pressure sensor of the present disclosure from respiration data received by another respiratory pressure sensor different in configuration from the respiratory pressure sensor of the present disclosure. In addition, the identification information may be identification information for identifying the respiration data acquired by the respiratory pressure sensor of the present disclosure and other data such as saturation of percutaneous oxygen (SpO₂) or vital signs information data acquired by a vital signs information sensor such as an ECG (electrocardiograph). In addition, the identification information may be identification information created by combining given patient information and the respiration data etc. in the present disclosure. The identification information may be set in advance by the external device 2 or the respiratory pressure sensor of the present disclosure or may be set with the external device 2 etc. by a medical worker handling the respiratory pressure sensor of the present disclosure.

The external device 2 is a device displaying information about the respiratory gas obtained from the subject. The external device 2 may be a device displaying the information about the respiratory gas and may have a controller (not shown) that analyzes the respiratory gas. In a case where the external device 2 has the controller, the controller may be configured to include a memory and a processor. The memory in the controller of the external device 2 may have the same configuration as or a similar configuration to that of the memory in the main body 4. The processor in the controller of the external device 2 may have the same configuration as or a similar configuration to that of the CPU 8. The external device 2 may be a patient monitor or display.

Next, an operation example of the respiratory pressure sensor will be described.

First, one end of a canula is connected to the input 3 in advance, and the other end of the canula is attached into the mouth or the nose of a subject. Respiratory gas from the mouth or the nose of the subject is guided from the attached cannula to the main body 4 through the input 3. The pressure sensor 6 of the main body 4 detects respiratory pressure from the guided respiratory gas.

The pressure sensor 6 outputs the detected respiratory pressure to the signal processor 7. The signal processor 7 generates respiration data corresponding to the respiratory pressure acquired from the pressure sensor 6, and outputs the respiration data to the A/D converter 81.

The A/D converter 81 receives the respiration data which is analog signal from the signal processor 7. The A/D converter 81 converts the analog respiration data to digital respiration data. The A/D converter 81 outputs the generated digital respiration data to the output 5.

When the output 5 is connected to the external device 2 or after that, the output 5 outputs the identification information to the external device 2. The external device 2 receives the identification information and identify that the respiration data received from the output 5 are recognizable data. After the identification information is output to the external device 2, the output 5 outputs the respiration data received from the A/D converter 81 to the external device 2.

According to the respiratory pressure sensor 1 of the present disclosure, as described above, the respiration data about the respiratory gas acquired from the subject are generated in real time, and the respiratory gas are outputted to the external device 2. Accordingly, a detection result of the respiratory gas can be analyzed in real time.

Respiration monitoring in the background art has been performed by an EtCO₂ (End Tidal CO₂) capnometer or a thoracic impedance system. Of them, the EtCO₂ capnometer is a device used for detection of carbon dioxide (CO₂) contained in respiratory gas, and has a matter that measurement may be inaccurate when the airway is not secured. In addition, the EtCO₂ capnometer acquires a measurement result of the respiratory gas as a curved line of a carbon dioxide concentration of expired gas (capnograph). Therefore, by use of the EtCO₂ capnometer, it is difficult to distinguish between hypopnea and hyperventilation and it is also impossible to measure a depth of respiration. The EtCO₂ capnometer is insufficient as the respiration monitoring in a non-intubation manner. Further, the EtCO₂ capnometer often uses a tracheal tube, and a sampling tube attached into the nose or the mouth. This may therefore give the subject a feeling of discomfort when the tracheal tube is attached into the subject. Consequently, there is room for improvement.

Of the respiration monitoring in the background art, the thoracic impedance system is a system in which an AC current is made to flow into a part having no action potential during detection of respiration, and a change of resistance inside the body of a subject is detected as a change of voltage. Therefore, the thoracic impedance system is weak to noise caused by movement of the body of the subject. In addition, the thoracic impedance system cannot distinguish a difference of respiratory motion between a normal time and an abnormal time. Therefore, the thoracic impedance system is insufficient as a respiration monitoring device.

According to the respiratory pressure sensor 1 of the present disclosure, the pressure sensor that detects respiratory pressure of respiratory gas, and the signal processor that applies signal processing to the respiratory pressure and generates respiration data are provided. Accordingly, a detection result of the respiratory gas can be analyzed in real time. In the background art, the respiration are cannot be analyzed by using the respiration data in real time.

Further, a complication relevant to upper airway obstruction accounts for a major proportion of respiratory complications immediately after surgery. In order to prevent the complications, it is important to check whether the upper airway obstruction is present or absent. However, it is impossible to check whether the upper airway obstruction is present or absent by the respiration monitoring method (the EtCO₂ capnometer or the thoracic impedance system) in the background art.

A screening test as to presence/absence of the upper airway obstruction is performed by a sleep apnea inspection device according to the background art. The screening test performed by the sleep apnea inspection device is to collect and analyze numerical values of intranasal pressure to thereby check whether upper airway obstruction of a subject is present or absent.

However, the screening test performed by the sleep apnea inspection device according to the background art is not to analyze the collected numerical values of the intranasal pressure in real time. There is no inspection device that can monitor presence/absence of upper airway obstruction in real time.

According to the respiratory pressure sensor 1 of the present disclosure, the pressure sensor that detects respiratory pressure of respiratory gas, and the signal processor that applies signal processing to the respiratory pressure and generates respiration data are provided. Accordingly, the respiration data can be analyzed in real time so that presence/absence of upper airway obstruction can be also analyzed in real time.

In addition, configuration may be also made so that the respiratory pressure sensor 1 of the present disclosure is additionally used with the EtCO₂ capnometer or the thoracic impedance system of the background art. In the thus made configuration in which the respiratory pressure sensor 1 of the present disclosure and the EtCO₂ capnometer or the thoracic impedance system according to the background art are used together, it is possible to perform respiration monitoring in a non-intubation manner, postoperative respiration management, respiration monitoring during endoscopic gastrointestinal tract surgery and respiration monitoring during nasal high-flow therapy. Accordingly, most suitable respiration monitoring in accordance with conditions of the subject can be provided as the configuration including respiration monitoring of respiratory pressure (intranasal pressure).

In addition, according to the respiratory pressure sensor 1 of the present disclosure, respiration data can be converted into a digital signal and outputted to the external device 2. Accordingly, the outputted respiration data can be less affected by noise than in a case where the respiration data are directly outputted as an analog signal. That is, the respiratory pressure sensor 1 of the present disclosure has noise immunity so that the respiratory pressure sensor 1 of the present disclosure can output the respiration data with less noise in comparison with a case where the respiration data are outputted as the analog signal.

In addition, according to the respiratory pressure sensor 1 of the present disclosure, respiration data outputted from the output 5 have identification information. Accordingly, labor, for example, for individually checking whether the respiration data outputted from the output 5 can be displayed on the external device 2 or not is unnecessary. In addition, even when a general-purpose connector having no identification information is used as the output, connection between the external device 2 and the output 5 cannot be established due to lack of the identification information. Accordingly, connection of a counterfeit product etc. with poor quality can be prevented.

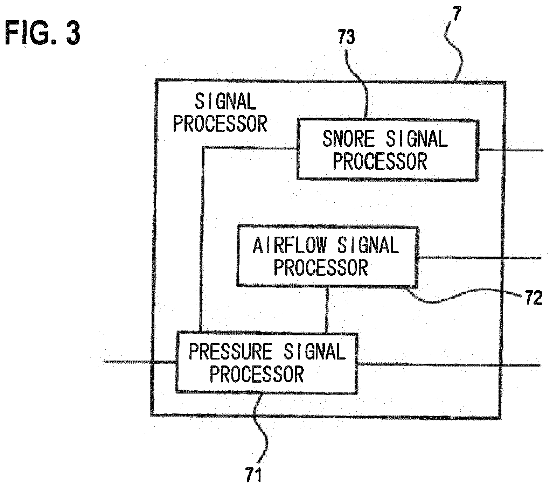

Incidentally, the signal processor 7 may be configured to generate respiratory pressure data and at least one of airflow data and snore data, as the respiration data. An example of an internal structure of the signal processor 7 is shown in Fig. 3. As shown in Fig. 3, the signal processor 7 includes a pressure signal processor 71, an airflow signal processor 72, and a snore signal processor 73.

The pressure signal processor 71 applies signal processing to respiratory pressure detected by the pressure sensor 6 and generates a respiration pressure signal. The respiratory pressure signal is not only outputted to the A/D converter 81 but also outputted to the airflow signal processor 72 and the snore signal processor 73.

The airflow signal processor 72 uses a filter for removing a high frequency component to extract an airflow component from the respiratory pressure signal so as to generate an airflow signal. The airflow signal is outputted to the A/D converter 81.

The snore signal processor 73 uses a filter for removing a low frequency component to extract a snore component from the respiratory pressure signal so as to generate a snore signal. The snore signal is outputted to the A/D converter 81. Incidentally, the snore signal may be a binary signal indicating snore presence or snore absence.

The signal processor 7 may be configured to include one of the airflow signal processor 72 and the snore signal processor 73. In addition, in Fig. 3, the snore signal processor 73 is configured to acquire the respiratory pressure from the pressure signal processor 71. However, the snore signal processor 73 is not limited to this configuration. The snore signal processor 73 may be configured to acquire the airflow signal from the airflow signal processor 72 so as to generate a snore signal based on the airflow signal.

According to the aforementioned configuration, the signal processor 7 generates the respiratory pressure signal and at least one of the airflow signal and the snore signal, as the respiration data. Accordingly, the signal processor 7 can generate the respiration data useful for real-time analysis of respiration disorder such as airway obstruction, apnea or hypopnea.

For example, the output 5 may be configured to output the respiration data to the external device 2 by wireless communication. The output 5 may be constituted, for example, by a wireless LAN card.

The output 5 may be configured to simultaneously output the identification information and the respiration data to the external device 2. The output 5 outputs the respiration data acquired from the A/D converter 81 to the external device 2, for example, together with the identification information held in the memory of the output 5. The external device 2 acquires the identification information to thereby identify that the respiration data acquired from the output 5 are recognizable data. Then, the external device 2 acquires the respiration data.

In addition, the identification information and the respiration data may be held in the respiratory pressure sensor 1 from which the external device 2 can acquire them, or may be held in a plurality of memories (not shown) inside the respiratory pressure sensor 1 separately. For example, the identification information may be held in the memory (not shown) inside the output 5 and the respiration data may be held in another memory (not shown) than the memory inside the output 5. In this case, configuration can be made so that the external device 2 first acquires the identification information held in the memory of the output 5 to thereby identify that the data can be recognized by the external device 2, and the external device 2 then acquires the respiration data held in the memory of the CPU 8.

In addition, as an example, the A/D converter 81 may be constituted by an electronic circuit having a sample hold circuit and a plurality of comparators. As an example, the signal processor 7 may be constituted by an analog electronic circuit formed on a board.

In addition, the CPU 8 may be configured to function as an analyzer that performs real-time analysis about the respiration data generated by the signal processor 7 and then generates analysis result data as a result of the real-time analysis. As an example, a program used by the analyzer for performing the analysis may be stored in a memory provided suitably inside the main body 4. The analysis result data may be outputted to the external device 2 etc. and generated so as to be able to be displayed on the external device 2 etc. in real time.

The CPU 8 serving as the analyzer can be configured to perform various kinds of analysis about respiratory gas, such as apnea, hypopnea, airway obstruction, respiration monitoring in a non-intubation manner, postoperative respiration management, respiration monitoring during endoscopic gastrointestinal tract surgery, respiration monitoring during nasal high-flow therapy, and upper airway obstruction.

According to the aforementioned configuration, the analyzer that analyzes the respiration data in real time to generate the analysis result data that can be displayed in real time is provided. Accordingly, it is possible to analyze a detection result about the respiratory gas in real time and it is possible to display the analysis result data in real time.

## Claims

1. A respiratory pressure sensor comprising:
an input configured to receive respiratory gas from at least one of a nasal cavity or an oral cavity of a subject;
a pressure sensor configured to detect respiratory pressure of the respiratory gas received from the input;
a signal processor configured to perform signal processing on the respiratory pressure detected by the pressure sensor and to generate respiration data; and
an output configured to output the respiration data to an external device,
wherein the signal processor is configured to generate a respiratory pressure signal and at least one of an airflow signal and a snore signal, as the respiration data, **characterised in that** the respiration data outputted from the output have identification information for identifying whether the external device can recognize the respiration data or not, and
the identification information is identification information for identifying the respiration data acquired by the respiratory pressure sensor and other vital signs information data, or identification information created by combining given patient information and the respiration data.

2. The respiratory pressure sensor according to claim 1, further comprising:
an A/D converter that performs digital conversion on the respiration data generated by the signal processor,
wherein the output is configured to output the respiration data on which the digital conversion is performed.

3. The respiratory pressure sensor according to any one of claim 1 to 2, further comprising an analyzer that analyzes the respiration data in real time to generate analysis result data that can be displayed in real time.

4. The respiratory pressure sensor according to claim 1,
wherein the output has a memory storing the identification information therein, and
wherein the output outputs the respiration data including the identification information stored in the memory.

## Patentansprüche

1. Drucksensor für Atemluftdruck, der umfasst:
einen Eingang, der so ausgeführt ist, dass er Atemgas aus einer Nasenhöhle oder/und einer Mundhöhle eines Patienten empfängt;
einen Drucksensor, der so ausgeführt ist, dass er Atemluftdruck des über den Eingang empfangenen Atemgases erfasst;
eine Signal-Verarbeitungseinrichtung, die so ausgeführt ist, dass sie Signalverarbeitung an dem durch den Drucksensor erfassten Atemluftdruck durchführt und Atmungs-Daten erzeugt; sowie
einen Ausgang, der so ausgeführt ist, dass er die Atmungs-Daten an eine externe Vorrichtung ausgibt, wobei die Signal-Verarbeitungseinrichtung so ausgeführt ist, dass sie ein Atemluftdruck-Signal sowie ein Luftstrom-Signal oder/und ein Schnarch-Signal als die Atmungs-Daten erzeugt,
**dadurch gekennzeichnet, dass** die über den Ausgang ausgegebenen Atmungs-Daten Identifizierungs-Informationen aufweisen, anhand derer identifiziert wird, ob die externe Vorrichtung die Atmungs-Daten erkennen kann, und
die Identifizierungs-Informationen Identifizierungs-Informationen, anhand derer die durch den Drucksensor für Atemluftdruck erfassten Atmungs-Daten oder andere Vitalzeichen-Informationsdaten identifiziert werden, oder Identifizierungs-Informationen sind, die durch Kombinieren gegebener Patienten-Informationen und der Atmungs-Daten erzeugt werden.

2. Drucksensor für Atemluftdruck nach Anspruch 1, der des Weiteren umfasst:
einen A/D-Wandler, der digitale Umwandlung an den durch die Signal-Verarbeitungseinrichtung erzeugten Atmungs-Daten durchführt,
wobei der Ausgang so ausgeführt ist, dass er die Atmungs-Daten ausgibt, an denen die digitale Umwandlung durchgeführt wird.

3. Drucksensor für Atemluftdruck nach einem der Ansprüche 1 bis 2, der des Weiteren eine Analyseeinrichtung umfasst, die die Atmungs-Daten in Echtzeit analysiert, um Analyseergebnis-Daten zu erzeugen, die in Echtzeit angezeigt werden können.

4. Drucksensor für Atemluftdruck nach Anspruch 1,
wobei der Ausgang einen Speicher aufweist, in dem die Identifizierungs-Informationen gespeichert werden, und
wobei der Ausgang die Atmungs-Daten einschließlich der in dem Speicher gespeicherten Identifizierungs-Informationen ausgibt.

## Revendications

1. Capteur de pression respiratoire comprenant :
une entrée configurée pour recevoir du gaz respiratoire d'au moins l'une parmi une cavité nasale ou une cavité buccale d'un sujet ;
un capteur de pression configuré pour détecter une pression respiratoire du gaz respiratoire reçu de l'entrée ;
un processeur de signal configuré pour exécuter un traitement de signal sur la pression respiratoire détectée par le capteur de pression et générer des données de respiration ; et
une sortie configurée pour délivrer les données de respiration vers un dispositif externe,
dans lequel le processeur de signal est configuré pour générer un signal de pression respiratoire et au moins l'un d'un signal de flux d'air et d'un signal de ronflement, comme données de respiration,
**caractérisé en ce que** les données de respiration délivrées depuis la sortie présentent des informations d'identification pour identifier si le dispositif externe peut reconnaître ou pas les données de respiration, et
les informations d'identification sont des informations d'identification pour identifier les données de respiration acquises par le capteur de pression respiratoire et d'autres données d'information sur des signes vitaux, ou informations d'identification créées en combinant des informations patient données et des données de respiration.

2. Capteur de pression respiratoire selon la revendication 1, comprenant en outre :
un convertisseur A/D qui exécute une conversion numérique sur les données de respiration générées par le processeur de signal,
dans lequel la sortie est configurée pour délivrer les données de respiration sur lesquelles la conversion numérique est exécutée.

3. Capteur de pression respiratoire selon l'une quelconque des revendications 1 à 2, comprenant en outre un analyseur qui analyse les données de respiration en temps réel pour générer des données de résultat d'analyse qui peuvent être affichées en temps réel.

4. Capteur de pression respiratoire selon la revendication 1,
dans lequel la sortie présente une mémoire stockant à l'intérieur des informations d'identification, et
dans lequel la sortie délivre les données de respiration incluant les informations d'identification stockées dans la mémoire.
